# EUROPÄISCHE PATENTSCHRIFT

(11) **EP 0 838 518 B1**
(45) Veröffentlichungstag und Bekanntmachung des Hinweises auf die Patenterteilung: **13.11.2002**
(21) Anmeldenummer: 98100877.4
(22) Anmeldetag: 03.09.1993
(51) Int. Cl.: C11D 1/835

(54) **Detergensgemische**
Detergent mixtures
Mélanges de détergents

(30) Priorität: 11.09.1992 US 943957
(43) Veröffentlichungstag der Anmeldung: 29.04.1998
(62) Teilanmeldung aus: 93919251.4
(73) Patentinhaber: Cognis Deutschland GmbH & Co. KG, 40589 Düsseldorf (DE)
(72) Erfinder: Obiols, Oriol Ponsati, Dr., 08005 Barcelona (ES); Gilabert, Nuria Bonastre, Dr., 08210 Barbeà del Vallès (ES)

(56) Entgegenhaltungen:
- EP-A- 0 106 692
- EP-A- 0 385 562
- EP-A- 0 387 064
- EP-A- 0 547 722
- WO-A-86/05509
- WO-A-87/02050
- WO-A-92/19693
- WO-A-92/22622
- WO-A-93/03130
- WO-A-93/10748

## Beschreibung

### Gebiet der Erfindung

Die Erfindung betrifft die Verwendung von Detergensgemischen, enthaltend Alk(en)yloligoglykoside und Esterquats zur Herstellung von Waschmitteln in flüssiger Form.

### Stand der Technik

Alkyloligoglucoside stellen nichtionische Tenside dar, die vollständig auf der Grundlage nachwachsender Rohstoffe - Zucker bzw. Stärke und Fettalkohol - aufgebaut sind. Neben ihren hervorragenden anwendungstechnischen Eigenschaften zeichnen sie sich durch eine besonders vorteilhafte ökotoxikologische Verträglichkeit aus, die sie für den Einsatz in einer Vielzahl von Anwendungsfeldem prädestiniert. Auch Mischungen von Alkyloligoglucosiden mit anderen, insbesondere anionischen Tensiden sind aus einer Vielzahl von Publikationen bekannt, von denen stellvertretend die Europäische Patentschrift **EP-B1 0070074** (Procter & Gamble) genannt werden soll. Detergensgemische, die neben Alkyloligoglucosiden kationische Tenside enthalten, werden ebenfalls in einer Reihe von Druckschriften beschrieben. In der **EP-B1 0094118** (Procter & Gamble) werden beispielsweise Zusammensetzungen für phosphatarme Waschmittel offenbart, die neben C₁₂-C₁₈-Alkyloligoglucosiden, Fettalkoholpolyglycolether und quartäre Ammoniumverbindungen enthalten. Gegenstand der Patentanmeldungen **EP-A1 0214285, EP-A1 0246246** (Staley Manuf.Co.) und **WO 87/02050** sind flüssige Desinfektionsmittel bzw. Feinwaschmittel enthaltend Alkyloligoglucoside und quartäre Ammoniumverbindungen. Gegenstand der **WO 93/03130** (Henkel) sind flüssige Waschmittel, die Alkoxylate verzweigter primärer Alkohole zusammen mit kationischen Tensiden u.a. vom Esterquat-Typ enthalten; daneben können noch Alkylglucoside anwesend sein. Aus der **EP-A1 0547772** (Colgate) sind wasserfreie Avivagemittel in Pulverform bekannt, die Harnstoff Esterquats und gegebenenfalls Alkylglucoside enthalten.

Aus der WO-A-93 03130, einen Dokument das unter den Art. 54(3) EPÜ fällt, sind flüssige Waschmittel bekannt, enthaltend Esterquats und Alkoxylate verzweigter Alkohole.

Aus der **DE-A1 3702287** (Colgate) sind schließlich Flüssigwaschmittel bekannt, die neben Alkyloligoglucosiden und quartären Ammoniumverbindungen noch weitere anionische und nichtionische Tenside enthalten. Obschon die Mittel des Stands der Technik in der Regel zufriedenstellende anwendungstechnische Ergebnisse liefern, ist ihre biologische Abbaubarkeit doch im Hinblick auf die ihnen gemeinsame Komponente - die quartären Ammoniumverbindungen (QAV's) - für eine Reihe von Anwendungen nicht ausreichend. Die Aufgabe der Erfindung hat folglich darin bestanden, neue Detergensgemische für die Herstellung von flüssigen Waschmitteln zur Verfügung zu stellen, die sich bei mindestens gleich guten anwendungstechnischen Eigenschaften durch eine verbesserte biologische Abbaubarkeit auszeichnen.

### Beschreibung der Erfindung

Gegenstand der Erfindung ist die Verwendung von Detergensgemischen, enthaltend
(a) Alkyl- und/oder Alkenyloligoglykoside der Formel **(I)**,

   **R**^{**1**}**O-[G]**_{**p**} **(I)**

   in der R¹ für Alkyl- oder Alkenylreste mit 1 bis 22 Kohlenstoffatomen, [G] für einen Zuckerrest mit 5 oder 6 Kohlenstoffatomen und p für Zahlen von 1 bis 10 steht, und
(b) Esterquats der Formel **(II)**, 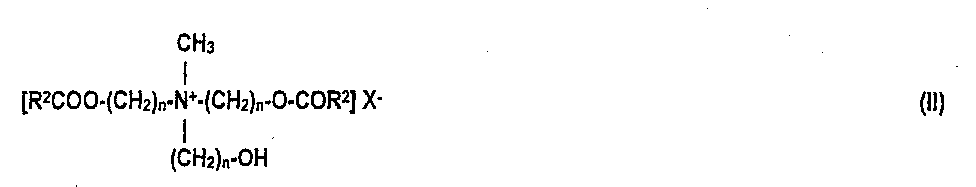 in der R²CO für einen aliphatischen Acylrest mit 12 bis 22 Kohlenstoffatomen und 0, 1, 2 oder 3 Doppelbindungen, n für 2 oder 3 und X für Halogenid, Methosulfat oder Methophosphat steht,
zur Herstellung von Flüssigwaschmitteln mit Avivageeigenschaften, mit der Maßgabe, dass die Komponenten (a) und (b) in Summe in Mengen von 10 bis 50 Gew.-% zugegeben und die Mittel frei von Alkoxylaten verzweigter primärer Alkohole sind.

Überraschenderweise wurde gefunden, daß die erfindungsgemäß zu verwendenden Detergensgemische nicht nur über hervorragende anwendungstechnische Eigenschaften verfügen, mit denen sie Produkten des Stands der Technik in vielen Fällen überlegen sind, sondern auch vollständig biologisch abbaubar und toxikologisch unbedenklich sind. Die Erfindung schließt die Erkenntnis ein, daß die Alkylund/oder Alkenyloligoglykoside die Solubilisierung der Esterquats in den unterschiedlichsten Medien unterstützen und insbesondere die Bildung von schwerlöslichen Salzen zwischen den Esterquats und gegebenenfalls mitverwendeten anionischen Tensiden weitgehend verhindern.

### Alkyl- und/oder Alkenyloligoglykoside

Alkyl- und oder Alkenyloligoglykosiden stellen bekannte Stoffe dar, die nach den einschlägigen Methoden der präparativen organischen Chemie erhalten werden können. Ein Verfahren zu ihrer Herstellung stellt beispielsweise die sauer katalysierte Acetalisierung von Glucose mit Fettalkoholen dar. Stellvertretend für das umfangreiche Schrifttum sei auf die Europäische Patentanmeldung **EP-A1 0301298** verwiesen. Bevorzugt sind Alkyl- und/oder Alkenyloligoglykoside, die sich von Aldosen bzw. Ketosen und wegen ihrer leichten Verfügbarkeit insbesondere von der Glucose ableiten. Die bevorzugten Alkyloligoglykoside sind somit die Alkyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkylund/oder Alkenyloligoglykosid eine analytisch ermittelte rechnerische Größe, die meistens eine gebrochene Zahl darstellt. Bevorzugt sind Alkyl- und/oder Alkenyloligoglykoside mit einem mittleren Oligomerisierungsgrad p von 1,1 bis 3,0; besonders bevorzugt sind solche Alkyl- und/oder Alkenyloligoglykoside, deren Oligomerisierungsgrad kleiner als 1,7 ist und insbesondere zwischen 1,2 und 1,4 liegt. Der Rest R¹ kann sich von gesättigten und/oder ungesättigten primären Alkoholen mit 1 bis 22, vorzugsweise 8 bis 10 bzw. 12 bis 18 Kohlenstoffatomen ableiten. Typische Beispiele sind Methanol, Butanol, Caprinalkohol, 2-Ethylhexanol, Caprylalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Arachylalkohol, Gadoleylalkohol, Behenylalkohol und Erucylalkohol sowie technische Schnitte, die diese Alkohole in unterschiedlichen Mengen enthalten können. Bevorzugt werden Alkyl- und/oder Alkenyloligoglykoside der Formel (I) einsetzt, in der R¹ für Alkylreste mit 8 bis 18 Kohlenstoffatomen, G für einen Glucoserest und p für Zahlen von 1 bis 3 steht.

### Esterquats

Unter Esterquats sind technische quaternierte Di-Fettsäuretrialkanolaminester-Salze zu verstehen, die nach den einschlägigen Methoden der präparativen organischen Chemie zugänglich sind. In diesem Zusammenhang sei auf den Übersichtsartikel von R.Puchta in **C.R. 3. CESIO, 1992, S.122f.** verwiesen. Zur Herstellung der Esterquats geht man üblicherweise von Fettsäuren aus, die im ersten Schritt mit Trialkanolaminen, wie beispielsweise Triethanol- oder Tripropanolamin verestert werden. Der gebildete Difettsäureester kann anschließend in an sich bekannter Weise beispielsweise mit Methylchlorid oder Dimethylsulfat quatemiert werden. Da es sich um technische Produkte handelt, sind in den Esterquats stets quaternierte Mono- und Triester als Nebenprodukte enthalten. Typische Beispiele für die Fettsäurekomponenten dieser Verbindungen sind Capronsäure, Caprylsäure, Caprinsäure, Laurinsäure, Myristinsäure, Palmitinsäure, Stearinsäure, Ölsäure, Elaidinsäure, Petroselinsäure, Linolsäure, Linolensäure, Arachinsäure, Gadoleinsäure, Behensäure und Erucasäure sowie deren technische Mischungen, wie sie beispielsweise bei der Spaltung von pflanzlichen Ölen oder tierischen Fetten anfallen. Bevorzugt sind Esterquats auf der Basis von Stearin- bzw. gehärteter Talgfettsäure in Form ihrer Chloride oder Methosulfate.

### Weitere Tenside

Neben den Alkyl- und/oder Alkenyloligoglykosiden und des Esterquats können die erfindungsgemäßen Detergensgemische noch weitere anionische, nichtionische sowie amphotere bzw. zwitterionische Tenside enthalten, z. B:
(a) **Anionische Tenside:** Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate (mit konventioneller und eingeengter Homologenverteilung), Glycerinethersulfate, Hydroxymischethersulfate, Monoglycerid(ether)sulfate, Fettsäureamid(ether)sulfate, Sulfosuccinate, Sulfosuccinamate, Sulfotriglyceride, Ethercarbonsäuren, Alkyloligoglucosidsulfate und/oder Alkyl(ether)phosphate.
(b) **Nichtionische Tenside:** Fettalkoholethoxylate (mit konventioneller und eingeengter Homologenverteilung), Polyolfettsäureester, Sorbitanester und/oder Polysorbate.
(c) **Amphotere bzw. zwitterionische Tenside:** Alkylamidobetaine, Aminopropionate, Aminoglycinate, Imidazoliniumbetaine und/oder Sulfobetaine.

Das Gewichtsverhältnis von Alkyl- und/oder Alkenyloligoglykosiden und Esterquats kann 5 : 95 bis 95 : 5, vorzugsweise 10 : 90 bis 90 : 10 und insbesondere 30 : 70 bis 70 : 30 betragen. Der Anteil weiterer Tenside an den Detergensgemischen kann dem gegenüber 1 bis 90, vorzugsweise 10 bis 80 und insbesondere 50 bis 70 Gew.-% - bezogen auf das Gemisch - ausmachen. Zur Herstellung der erfindungsgemäßen Detergensgemische ist es ausreichend, die Komponenten, gegebenenfalls unter Erwärmung auf 30 bis 40°C, zu vermischen und - falls erforderlich - zu homogenisieren. Dabei ist sowohl möglich, von Konzentraten auszugehen, die mit Wasser auf eine Anwendungskonzentration von 1 bis 50 vorzugsweise 15 bis 30 Gew.-% verdünnt werden oder gleich verdünnte, wäßrige Ausgangsstoffe einzusetzen. In jedem Fall handelt es sich um einen rein mechanischen Vorgang; eine chemische Reaktion findet nicht statt.

### Gewerbliche Anwendbarkeit

Die erfindungsgemäßen Detergensgemische zeichnen sich durch ausgezeichnete Wasch- und Reinigungsleistung, Textilavivage und antistatische Ausrüstung von Fasern sowie hohe ökotoxikologische Verträglichkeit aus.

Flüssigwaschmittel, insbesondere flüssige Feinwaschmittel, auf Basis der erfindungsgemäßen Detergensgemische, die diese in Mengen von 10 bis 50 Gew.-% enthalten, können als Hilfs- und Zusatzstoffe beispielsweise Builder, Salze, Bleichmittel, Bleichaktivatoren, optische Aufheller, Vergrauungsinhibitoren, Lösungsvermittler, Entschäumer und Enzyme aufweisen. Übliche Builder sind Natriumaluminiumsilicate (Zeolithe), Phosphate, Phosphonate, Ethylendieamintetraessigsäure, Nitrilotriacetat, Citronensäure und/oder Polycarboxylate. Als Salze bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

## Patentansprüche

1. Verwendung von Detergensgemischen, enthaltend
(a) Alkyl- und/oder Alkenyloligoglykoside der Formel **(I),**
**R**^{**1**}**O-[G]**_{**p**} **(I)**
in der R¹ für Alkyl- oder Alkenylreste mit 1 bis 22 Kohlenstoffatomen, [G] für einen Zuckerrest mit 5 oder 6 Kohlenstoffatomen und p für Zahlen von 1 bis 10 steht, und
(b) Esterquats der Formel **(II),** 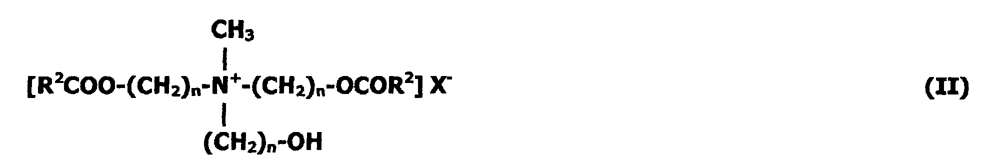 in der R²CO für einen aliphatischen Acylrest mit 12 bis 22 Kohlenstoffatomen und 0, 1, 2 oder 3 Doppelbindungen, n für 2 oder 3 und X für Halogenid, Methosulfat oder Methophosphat steht,
zur Herstellung von Flüssigwaschmitteln mit Avivageeigenschaften, mit der Maßgabe, dass die Komponenten (a) und (b) in Summe in Mengen von 10 bis 50 Gew.-% zugegeben und die Mittel frei von Alkoxylaten verzweigter primärer Alkohole sind.

2. Verwendung nach Anspruch 1, **dadurch gekennzeichnet, dass** man die Detergensgemische zusammen mit anionischen, nichtionischen und/oder amphoteren bzw. zwitterionischen Tensiden einsetzt.

3. Verwendung nach Anspruch 2, **dadurch gekennzeichnet, dass** man die Detergensgemische zusammen mit anionischen Tensiden einsetzt, die ausgewählt sind aus der Gruppe, die von Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Hydroxymischethersulfaten, Monoglycerid(ether)sulfaten, Fettsäureamid(ether)sulfaten, Sulfosuccinaten, Sulfosuccinamaten, Sulfotriglyceriden, Ethercarbonsäuren, Alkyloligoglucosidsulfaten und Alkyl(ether)phosphaten gebildet wird.

4. Verwendung nach Anspruch 2, **dadurch gekennzeichnet, dass** man die Detergensgemische zusammen mit nichtionischen Tensiden einsetzt, die ausgewählt sind aus der Gruppe, die ausgewählt sind aus der Gruppe, die von Fettalkoholethoxylaten, Polyolfettsäureestern, Sorbitanestern und Polysorbaten gebildet wird.

5. Verwendung nach Anspruch 2, **dadurch gekennzeichnet, dass** man die Detergensgemische zusammen mit amphoteren bzw. zwitterionischen Tensiden einsetzt, die ausgewählt sind aus der Gruppe, die von Alkylamidobetainen , Aminopropionaten, Aminoglycinaten, Imidazoliniumbetainen und Sulfobetainen gebildet wird.

6. Verwendung nach den Ansprüchen 1 bis 5, **dadurch gekennzeichnet, dass** man die Alkylund/oder Alkenyloligoglykosiden und Esterquats im Gewichtsverhältnis 5 : 95 bis 95 : 5 einsetzt.

## Claims

1. The use of detergent mixtures containing
(a) alkyl and/or alkenyl oligoglycosides corresponding to formula **(I):**
**R**^{**1**}**O-[G]**_{**p**} **(I)**
in which R¹ represents alkyl or alkenyl groups containing 1 to 22 carbon atoms, [G] is a sugar unit containing 5 or 6 carbon atoms and p is a number of 1 to 10, and
(b) esterquats corresponding to formula **(II):** 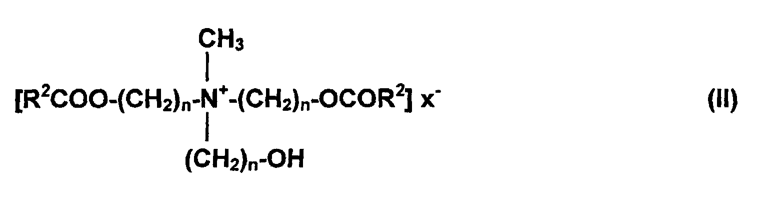 in which R²CO is an aliphatic acyl group containing 12 to 22 carbon atoms and 0, 1, 2 or 3 double bonds, n = 2 or 3 and X is halide, methosulfate or methophosphate,
for the production of liquid detergents with softening properties, with the proviso that components (a) and (b) are present in total quantities of 10 to 50% by weight and the detergents are free from alkoxylates of branched primary alcohols.

2. The use claimed in claim 1, **characterized in that** the detergent mixtures are used together with anionic, nonionic and/or amphoteric or zwitterionic surfactants.

3. The use claimed in claim 2, **characterized in that** the detergent mixtures are used together with anionic surfactants selected from the group consisting of alkyl benzenesulfonates, alkanesulfonates, olefin sulfonates, alkyl ether sulfonates, glycerol ether sulfonates, α-methylester sulfonates, sulfofatty acids, alkyl sulfates, fatty alcohol ether sulfates, glycerol ether sulfates, hydroxy mixed ether sulfates, monoglyceride (ether) sulfates, fatty acid amide (ether) sulfates, sulfosuccinates, sulfosuccinamates, sulfotriglycerides, ether carboxylic acids, alkyl oligoglucoside sulfates and alkyl (ether) phosphates.

4. The use claimed in claim 2, **characterized in that** the detergent mixtures are used together with nonionic surfactants selected from the group consisting of fatty alcohol ethoxylates, polyol fatty acid esters, sorbitan esters and polysorbates.

5. The use claimed in claim 2, **characterized in that** the detergent mixtures are used together with amphoteric or zwitterionic surfactants selected from the group consisting of alkyl amidobetaines, aminopropionates, aminoglycinates, imidazolinium betaines and sulfobetaines.

6. The use claimed in claims 1 to 5, **characterized in that** the alkyl and/or alkenyl oligoglycosides and esterquats are used in a ratio by weight of 5:95 to 95:5.

## Revendications

1. Utilisation de mélanges de détergents contenant :
a) des alkyl et/ou alkényloligoglycosides de formule (I)
R¹O-[G]ₚ (I)
dans laquelle R¹ représente des restes alkyle ou alkényle ayant de 1 à 22 atomes de carbone, [G] représente un reste de sucre ayant 5 ou 6 atomes de carbone et p représente des nombres allant de 1 à 10, et
b) des esterquats de formule (II) 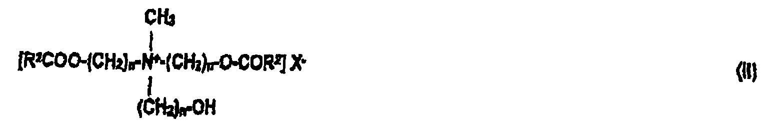 dans laquelle R²CO représente un reste acyle aliphatique ayant de 12 à 22 atomes de carbone, et 0, 1, 2, ou 3 double liaisons, n représente 2 ou 3 et X représente un halogénure, un méthosulfate ou un méthophosphate,
en vue de la préparation de produits de lavage liquides ayant des propriétés d'avivage, avec la restriction que les composants a) et b) sont présents globalement en quantités de 10 à 50 % en poids et que les produits sont exempts d'alkoxylates d'alcools primaires ramifiés.

2. Utilisation selon la revendication 1,
**caractérisé en ce qu'**
on met en oeuvre les mélanges de détergents conjointement avec des agents tensioactifs anioniques, non ioniques et/ou amphotères ou zwitterioniques.

3. Utilisation selon la revendication 2,
**caractérisée en ce qu'**
on met en oeuvre les mélanges de détergents conjointement avec des agents tensioactifs anioniques choisis dans le groupe formé par les alkylbenzènesulfonates, les alkanesulfonates, les oléfinesulfonates, les alkyléthersulfonates, les glycéroléthersulfonates, sulfonates d'ester en α de méthyle, les acides gras sulfonés, les sulfates d'alcyle, les sulfates d'éther d'alcool gras, les sulfates d'éther de glycérol, les sulfates, d'éther mixtes hydroxylés, les sulfates d'(éther) de monoglycéride, les sulfates (d'éther) d'amide d'acide gras, les sulfosuccinates, les sulfosuccinamates, les sulfotriglycérides, les acides éther carboxyliques, les sulfates d'alkyloligoglucosides et les phosphates (d'éther) d'alkyle.

4. Utilisation selon la revendication 2,
**caractérisée en ce qu'**
on met en oeuvre les mélanges de détergents conjointement avec les agents tensioactifs non ioniques qui sont choisis dans le groupe formé parmi les éthoxylates d'alcool gras, les esters d'acide gras et de polyol, les esters de sorbitane et les polysorbates.

5. Utilisation selon la revendication 2,
**caractérisée en ce qu'**
on met en oeuvre les mélanges de détergents conjointement avec des agents tensioactifs amphotères ou zwitterioniques qui sont choisis dans le groupe formé des alkylamidobétaïnes, des aminopropionates, des aminoglycinates, des bétaïnes d'imidazolinium et des sulfobétaïnes.

6. Utilisation selon l'une quelconque des revendications 1 à 5,
**caractérisée en ce qu'**
on met en oeuvres les alkyl- et/ou alkényloligoglycosides et les esterquats dans un rapport en poids de 5 :95 à 95 :5.
